(19) Europäisches Patentamt
European Patent Office
Office européen des brevets

(11) Publication number: 0 334 369 B1

# (12) EUROPEAN PATENT SPECIFICATION

(45) Date of publication of patent specification: 23.06.93

(51) Int. Cl.5: A61K 31/415, A61K 31/425, A61K 31/44

(21) Application number: 89105270.6

(22) Date of filing: 23.03.89

## (54) Antiarteriosclerotic agent.

(30) Priority: 24.03.88 JP 70453/88

(43) Date of publication of application:
27.09.89 Bulletin 89/39

(45) Publication of the grant of the patent:
23.06.93 Bulletin 93/25

(84) Designated Contracting States:
BE CH DE FR GB IT LI NL SE

(56) References cited:

PATENT ABSTRACTS OF JAPAN, vol. 11, no. 13 (C-397)(2460), 14 January 1987*

THROMB. RES., vol. 47, no. 4, 15 August 1987; M.UDVARDY et al., pp. 479-484*

THROMB. RES., vol. 44, no. 2, 15 October 1986; J.MOSCAT et al., pp. 197-205*

THE MERCK MANUAL, 15th ed., 1987, R.Berkow, MD (Ed.), Merck Sharp & Dohme Research Laboratories, Rahway, NJ (US)*

(73) Proprietor: DAIICHI PHARMACEUTICAL CO., LTD.
14-10, Nihonbashi 3-chome
Chuo-ku, Tokyo 103(JP)

(72) Inventor: Ishimitsu, Toshihiko
9-13-307, Yushima 4-chome Bunkyo-ku
Tokyo(JP)
Inventor: Matsuoka, Horoaki
1-5-903, Arai 1-chome Nakano-ku
Tokyo(JP)
Inventor: Sugimoto, Tsuneaki
29-10, Shimouma 5-chome Setagaya ku
Tokyo(JP)
Inventor: Ishii, Masao
39-27, Hikawadai 2-chome
Higashikurume-shi Tokyo(JP)
Inventor: Shiozawa, Tomoo
Daiichi Seiyaku Co., Ltd. 8-5, Hatchobori 2-chome
Chuo-ku Tokyo(JP)

(74) Representative: Kinzebach, Werner, Dr. et al
Patentanwälte Reitstötter, Kinzebach und Partner Sternwartstrasse 4 Postfach 86 06 49
W-8000 München 86 (DE)

EP 0 334 369 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of a compound represented by the formula (I):

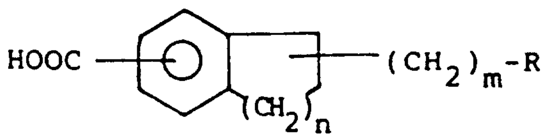

(I)

wherein R represents an imidazolyl group, a thiazolyl group, or a pyridyl group; n represents 1 or 2; and m represents an integer of from 1 to 4,
or a salt thereof for preparing a pharmaceutical composition for treating arteriosclerosis.

It is for example disclosed in JP 61-191612 (A) that compounds of formula I may be applied in the treatment of ischemic cardiac disease. It is further disclosed that these compounds suppress the synthesis of thromboxane $A_2$.
In Throm. Res. (1987) 47, 479 - 484 it is mentioned that thromboxane $A_2$ synthesized in platelets causes platelet aggregation, vasoconstriction and elevation of the blood pressure.
In Throm. Res. (1986) 44, 197-205 thromboxane $A_2$ is disclosed as potent homeostatic regulator with proaggregating, thrombotic and vasoconstrictor properties.

The compounds represented by the formula (I) are however unknown for their antiarteriosclerotic activity.

Preventing and treating effects on ischemic heart diseases are not related with antiarteriosclerotic activity.

As a result of intensive and extensive investigations, the inventors have found that the compounds of the formula (I) exhibit antiarteriosclerotic activity. More specifically, it was found that compounds of formula (I) significantly suppress the proliferation of vascular smooth muscle cells.

Based on this invention an antiarteriosclerotic agent containing the compound represented by the formula (I) or a salt thereof as an active ingredient can be provided which upon administration suppresses the proliferation of vascular smooth muscle cells.

The salts of the compounds of the formula (I) which can be used in the present invention include acid addition salts formed with inorganic acids (e.g., hydrochloric acid, sulfuric acid, and nitric acid) or organic acids (e.g., fumaric acid, tartaric acid, maleic acid, succinic acid, and oxalic acid); and alkali metal salts or alkaline earth metal salts formed from the carboxyl group and alkali metals (e.g., sodium and potassium) or alkaline earth metals (e.g., calcium and magnesium).

The compounds of the formula (I) and salts thereof were tested for acute toxicity in rats (p.o.) and, as a result, proved highly safe.

The compounds of the formula (I) and salts thereof can be formulated in various pharmaceutical preparations, such as tablets, powders, capsules and injectable solutions, by known pharmaceutical techniques. The compounds of the formula (I) or salts thereof are usually administered orally, subcutaneously, intramuscularly, or intravenously.

The dose level of the compounds of the formula (I) or salts thereof usually ranges from about 50 to about 1,000 mg/day/adult (body weight: about 60 kg) in oral administration.

As demonstrated in Test Examples hereinafter given, the compounds of formula (I) and salts thereof significantly suppressed a proliferation of vascular smooth muscle culture cells and an uptake of tritium-labeled thymidine into DNA (hereinafter referred to as $^3$H-TU) in spontaneous hypertensive rats (SHR) which are important indices for progress of arteriosclerosis. The compounds of formula (I) and the salts thereof are therefore excellent as antiarteriosclerotic agents.

The present invention is now illustrated in greater detail by way of the following Test Examples.

### TEST EXAMPLE 1

Effects on Proliferation of Vascular Smooth Muscle Culture Cells

Vascular smooth muscle cells (hereinafter referred to as VSMC) were separated from the aorta of seven 7-week-old male SHR and cultured according to the explant method [cf. Ross, R.J. Cell Biol., Vol. 50, 172 (1971)]. Culture cells of passage Nos. 4 to 10 were used for testing.

As a test compound, 6-(1-Imidazolylmethyl)-5,6,7,8-tetrahydro-2-naphthalenecarboxylic acid hydrochloride 1/2 hydrate (hereinafter referred to as Compound A) was used.

The proliferation ability of VSMC was evaluated in terms of time required for doubling the number of the cells (hereinafter referred to as DT) and $^3$H-TU according to the following test methods.

1) Determination of DT:

VSMC (about 5 x $10^4$ cells) were cultured on a plate for 24 hours. Compound A was added to the culture, and cultivation was continued for an additional period of 48 hours. The number of cells before the addition of Compound A and after the 48-hour cultivation were counted by means of a light microscope, and DT was obtained therefrom by the equation:

$$DT = (t_1 - t_2) \times \frac{\log 2}{\log N_1 - \log N_2}$$

wherein $t_1$ is the time at which the number of cells before addition of Compound A is counted; $t_2$ is the time at which the number of cells after addition of Compound A is counted; $N_1$ is the number of cells at $t_1$; and $N_2$ is the number of cells at $t_2$.

2) Determination of $^3$H-TU:

VSMC (about $10^4$ cells) was inoculated to a 24-well polystyrene dish and cultured for 24 hours. The culture medium was exchanged with a medium containing Compound A, and 0.25 $\mu$Ci of $^3$H-thymidine (20 to 30 Ci/mmol) was added thereto. After culturing for 24 hours, the culture was repeatedly washed with Dulbecco-PBS and treated with a 5% perchloric acid aqueous solution. The radioactivity of the acid-insoluble fraction was determined by the use of a scintillator.

The results of these tests are shown in Tables 1 and 2 below.

TABLE 1

| DT (hour) | |
|---|---|
| Control Group | Treated Group |
| 24.32 | 26.16 |
| 27.41 | 27.81 |
| 26.50 | 26.59 |
| 27.05 | 28.94 |
| 20.70 | 21.66 |
| 23.44 | 24.41 |
| P < 0.02 vs. control group by Student's t-test | |

TABLE 2

| $^3$H-TU (x $10^{-3}$ dpm/$10^4$ cells/24 hrs) | |
|---|---|
| Control Group | Treated Group |
| 18.79 | 18.05 |
| 22.30 | 20.80 |
| 11.51 | 11.51 |
| 21.35 | 19.62 |
| 17.06 | 16.68 |
| 14.64 | 12.43 |
| P < 0.03 vs. control group by Student's t-test | |

SHR is one of important experimental models of arteriosclerosis. Thickening of the vascular smooth muscle, i.e., proliferation of the vascular smooth muscle cells, is one of the important indications of causes and progresses of arteriosclerosis.

As is apparent from Tables 1 and 2, the compound of formula (I) significantly reduced DT and $^3$H-TU of the culture VSMC of SHR, i.e., suppressed the proliferation of VSMC. Accordingly, it was confirmed that the compound of formula (I) is excellent as an anti-arteriosclerotic agent.

TEST EXAMPLE 2

Acute toxicity of the compound A in rats (p.o.) is shown in Table 3.

TABLE 3

| Acute Toxicity (rat, p.o.) $^{LD}50$ (mg/kg) | |
|---|---|
| Male | Female |
| 2438 | 1994 |

## Claims

**1.** The use of at least one compound represented by the formula (I)

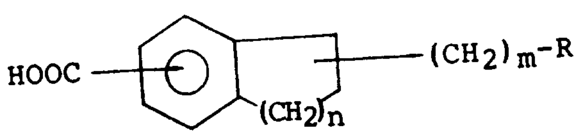

(I)

wherein R represents an imidazolyl group, a thiazolyl group, or a pyridyl group; n represents 1 or 2; and m represents an integer of from 1 to 4,
or a salt thereof for preparing a pharmaceutical composition for suppression of proliferation of vascular smooth muscle cells.

**2.** The use of at least one compound represented by the formula (I) as defined in claim 1
or a salt thereof for preparing a pharmaceutical composition for treating arteriosclerosis based on suppression of proliferation of vascular smooth muscle cells.

**Patentansprüche**

1. Verwendung wenigstens einer Verbindung der Formel (I)

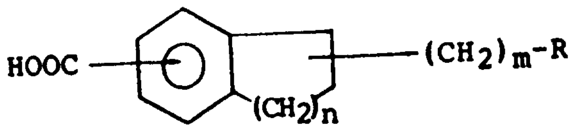

(I)

worin R für eine Imidazolylgruppe, Thiazolylgruppe oder Pyridylgruppe steht; n für 1 oder 2 steht; und m für eine ganze Zahl von 1 bis 4 steht, oder eines Salzes davon zur Herstellung eines pharmazeutischen Mittels zur Suppression der Proliferation vaskulärer glatter Muskelzellen.

2. Verwendung wenigstens einer Verbindung der in Anspruch 1 definierten Formel (I) oder eines Salzes davon zur Herstellung eines pharmazeutischen Mittels zur Behandlung von Arteriosklerose, die auf der Suppression der Proliferation vaskulärer Muskelzellen basiert.

**Revendications**

1. Utilisation d'au moins un composé représenté par la formule (I)

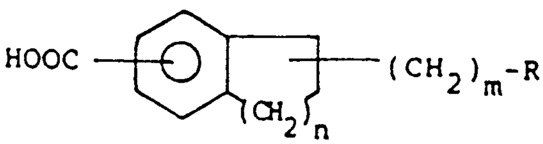

(I)

dans laquelle R représente un groupement imidazolyle, un groupement thiazolyle ou un groupement pyridyle; n vaut 1 ou 2 ; et m représente un entier compris entre 1 et 4,
ou un sel de celui-ci pour la préparation d'une composition pharmaceutique pour la suppression de la prolifération des cellules musculaires lisses vasculaires.

2. Utilisation d'au moins un composé représenté par la formule (I) définie comme dans la revendication 1 ou d'un de ses sels pour la préparation d'une composition pharmaceutique pour le traitement de l'artériosclérose basée sur la suppression de la prolifération des cellules musculaires lisses vasculaires.